(19) Europäisches Patentamt
European Patent Office
Office européen des brevets

(11) EP 0 712 858 A1

(12) **DEMANDE DE BREVET EUROPEEN**

(43) Date de publication:
**22.05.1996 Bulletin 1996/21**

(21) Numéro de dépôt: **95420311.3**

(22) Date de dépôt: **14.11.1995**

(51) Int Cl.[6]: **C07H 15/06**, C07H 15/04, C07H 15/12, C07H 13/04, C07C 235/10, C11D 1/00, A61K 9/127, B01F 17/56

(84) Etats contractants désignés:
**AT BE CH DE DK ES FR GB GR IE IT LI LU MC NL PT SE**

(30) Priorité: **17.11.1994 FR 9413992**

(71) Demandeur: **STEPAN EUROPE**
**F-38340 Voreppe (FR)**

(72) Inventeurs:
- **Caparros, Alain**
  **F-31600 Muret (FR)**
- **Brisset, Florence**
  **F-31130 Balma (FR)**
- **Rico-Lattes, Isabelle**
  **F-31650 Auzielle (FR)**
- **Lattes, Armand**
  **F-31650 Auzielle (FR)**
- **Godefroy, Lionel**
  **F-38430 Moirans (FR)**

(74) Mandataire: **Guerre, Dominique et al**
**Cabinet Germain et Maureau**
**20 Boulevard Eugène Deruelle**
**BP 3011**
**F-69392 Lyon Cédex 03 (FR)**

(54) **Composés amphiphiles mixtes**

(57) Composé chimique bolaamphiphile ou bolaforme, comportant une chaîne carbonée hydrophobe, linéaire ou ramifiée, aux extrémités de laquelle sont disposées deux parties ou têtes hydrophiles, ledit composé répondant à la formule générale suivante:

R A E Y

dans laquelle :

- E représente la chaîne carbonée, interrompue éventuellement par au moins un groupement aromatique, hétérocyclique, ou fonctionnel
- A est un groupe fonctionnel
- R est un résidu d'un glucide réducteur à chaîne linéaire ou cyclisée, ou un dérivé du glucide réducteur, lié directement ou indirectement par une de ses fonctions aldéhyde ou cétone, ou dérivée, au groupe fonctionnel A,
- Y représente une tête hydrophile,

**caractérisée en ce que** Y comprend au moins un groupe anionique choisi parmi les ions carboxylate, sulfate, phosphate, sulfonate, phosphonate, Y étant lié directement ou indirectement à la chaîne carbonée E.

EP 0 712 858 A1

## Description

L'invention se rapporte à de nouveaux composés dits bolaamphiphiles ou bolaformes, présentant des propriétés tensio-actives, et utilisables notamment comme agents vésiculaires.

Par composés bolaamphiphiles ou bolaformes, on entend tout composé comportant :

- au moins une chaîne carbonée relativement longue, linéaire ou ramifiée, et hydrophobe, avec éventuellement des hétéroatomes
- et deux parties polaires ou têtes hydrophiles, disposées et liées par covalence respectivement aux deux extrémités de la chaîne carbonée.

Selon la demande EP-0 541 467 au nom de la Demanderesse, dont le contenu est incorporé à la présente demande de brevet en tant que de besoin, il est décrit des composés bolaformes répondant à la formule générale suivante :

R A E A R

dans laquelle :

- E représente une chaîne carbonée, comportant au moins six atomes de carbone, interrompue éventuellement par au moins un groupement aromatique, ou hétérocyclique, ou fonctionnel
- A est un groupe fonctionnel de liaison
- R est un résidu d'un glucide réducteur à chaîne linéaire ou cyclisée, ou d'un dérivé dudit glucide réducteur, lié directement ou indirectement (par exemple par une chaîne alkyle) par une de ses fonctions aldéhyde ou cétone, ou dérivée (par exemple amide), au groupe fonctionnel A.

Les composés précités trouvent des applications comme agents tensioactifs, notamment pour former directement des structures vésiculaires, applications d'autant plus nombreuses qu'ils ne présentent pas ou peu de toxicité.

On connaît aussi des composés bolaformes présentant des têtes anioniques, identiques ou non, du type sulfate ou carboxylate, séparées par une ou deux chaînes hydrocarbonées, tels que les composés décrits par J.H. FUHRHOP et al, dans J. Am. Chem. Soc., 1986, 108, 1785. Ces derniers composés ne sont aujourd'hui pas utilisés dans l'industrie, en raison de leur synthèse difficile dont le nombre d'étapes et le rendement faible de chacune d'elles, les rendant totalement inadaptés à une grande production industrielle.

C'est en recherchant à améliorer les propriétés tensioactives de composés bolaformes à double tête sucre, que la Demanderesse a élaboré une nouvelle famille de composés bolaformes, permettant à la fois de conserver les effets déjà connus des composés à double tête sucre, tout en apportant des qualités complémentaires, notamment quant à leur solubilité dans l'eau et leur capacité à former des vésicules stables.

C'est ainsi qu'un premier objet de l'invention est un composé bolaforme dit mixte, c'est-à-dire à deux têtes de nature différente, dont les propriétés en solution peuvent être adaptées, en fonction du pH, à l'utilisation recherchée, et qui permet en outre d'obtenir des vésicules unilamellaires.

Selon US-3 855 290, on connaît des composés bolaformes mixtes comprenant une tête polyol et un tête cationique, décrits à titre d'agent émolliant, dans une composition cosmétique. De telles propriétés n'infèrent pas nécessairement des propriétés tensioactives et peuvent être apportées par des produits bien différents des détergents, tels que des huiles.

Un composé de la présente invention répond à la formule générale suivante, (I) :

R A E Y

dans laquelle :

- E représente une chaîne carbonée, interrompue éventuellement par au moins un groupement aromatique, hétérocyclique, ou fonctionnel
- A est un groupe fonctionnel
- R est un résidu d'un glucide réducteur à chaîne linéaire ou cyclisée, ou d'un dérivé dudit glucide réducteur, lié directement ou indirectement par une de ses fonctions aldéhyde ou cétone, ou dérivée, au groupe fonctionnel A
- Y représente une tête hydrophile, comprenant au moins un groupe anionique choisi parmi les ions carboxylate, sulfate, sulfonate, phosphate et phosphonate, Y étant lié directement ou indirectement à la chaîne carbonée E.

Le composé décrit ci-dessus possède une tête non ionique, la tête sucre représentée par le groupe R, et une tête ionique représentée par le groupe Y, et la combinaison de ces deux têtes conduit à des composés parfaitement hydrosolubles, quel que soit le pH, et apportent une solution au problème de stabilité des vésicules unilamellaires obtenues à partir de ces composés.

En effet, les bolaformes à deux têtes non ioniques forment en solution des vésicules parfois instables sous forme de microvésicules, par coalescence. Leurs diamètres sont alors trop élevés pour conférer des propriétés intéressantes à la solution.

Au contraire, les composés de l'invention, grâce à l'effet de charge obtenu par l'existence sur une même molécule d'une tête chargée et d'une tête non chargée forment des vésicules, aussi bien des macrovésicules que des microvésicules, sans que n'intervienne le phénomène de coalescence.

Au surplus, on a découvert que certains des composés de l'invention présentaient des propriétés moussantes.

Selon l'invention, le résidu de glucide réducteur représenté par R dans la formule I, peut être un monosaccharide notamment choisi parmi le glucose et le galactose, ou un polysaccharide, tels que ceux du groupe comprenant le lactose, le cellobiose et le maltose.

La chaîne carbonée E de la formule I est avantageusement une chaîne carbonée saturée, comprenant de préférence jusqu'à 12 atomes de carbone.

Le groupe fonctionnel A est préférentiellement choisi parmi les fonctions O, OCO, S, NH, NHCO.

Dans les composés de l'invention, la tête ionique représentée par Y dans la formule I, est liée soit directement à la chaîne carbonée E, soit indirectement, et dans ce dernier cas, un groupe fonctionnel, avantageusement choisi parmi les fonctions O, OCO, S, NH et NHCO, lie E à Y.

L'utilisation de composés de l'invention est particulièrement adaptée à la préparation de compositions tensioactives et d'agents vésiculaires, incorporant respectivement au moins un composé de l'invention.

Un autre avantage principal de l'invention réside dans la facilité d'accès aux composés décrits, selon le procédé suivant: on fait réagir une forme oxydée, ou réduite, ou aminée du glucide réducteur, avec un produit répondant à la formule B E Y', B correspondant au groupe fonctionnel A avant condensation avec la forme réactive du glucide, et Y' correspondant à la forme non chargée du groupe Y.

La forme oxydée du glucide réducteur est par exemple la forme lactone ou acide.

Le composé de formule I dans laquelle R représente le glucose ou le galactose est avantageusement obtenu à partir de la forme lactone et en particulier la gluconolactone.

Un composé de l'invention répondant à la formule I dans laquelle R représente le lactose, le cellobiose ou le maltose est de préférence obtenu à partir de la forme oxydée du glucide réducteur consistant par exemple en l'acide lactobionique.

Selon l'invention, le composé de formule I dans laquelle Y est un groupe anionique carboxylate est obtenu par mise en réaction de la forme oxydée du sucre réducteur, et notamment la gluconolactone ou l'acide lactobionique, avec un acide aminé répondant à la formule BEY', dans laquelle Y' correspond à la forme YH et représente la fonction acide dudit acide aminé, en présence de soude.

La présente invention est à présent décrite de manière plus détaillée dans les Exemples 1 à 10 et dans le dessin composé de deux photographies, d'où ressortiront les avantages des compositions de l'invention.

La figure 1 est une photomicrographie de vésicules de carboxylate de gluconamidodécane (concentration: $5.10^{-2}$M, grossissement: x72000), obtenue par microscopie électronique.

La figure 2 est une photomicrographie de vésicules de carboxylate de gluconamidodécane (concentration: $5.10^{-2}$M, grossissement: x91000), également obtenue par microscopie électronique.

**Exemple 1:** Préparation du carboxylate de gluconoamidoheptane

A une solution de soude (2,7 mmoles: 0,110 g) dans le méthanol (50 ml) sont ajoutés l'acide amino-8-caprylique (2,7 mmoles: 0,437 g) puis la δ-gluconolactone (2,7 mmoles: 0,490 g).

Le milieu réactionnel est agité pendant 24 heures à température ambiante. Le solvant est évaporé sous vide puis le produit est lyophilisé.

On obtient 0,97 g du composé de formule suivante:

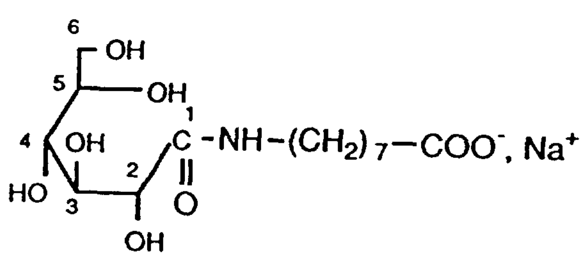

Ce produit obtenu avec un rendement de 99% a été caractérisé par RMN $^{1}H$ et RMN $^{13}C$, spectroscopie de masse et une analyse centésimale.

**RMN $^{13}C$, DMSO $d_6$, δ ppm:**

Chaîne alkyle: $-NH-CH_2-CH_2-CH_2-CH_2-CH_2-CH_2-CH_2-COO^-$

$\alpha \quad \beta \quad \gamma \quad \delta \quad \varepsilon \quad \zeta \quad \eta$

(25,69; 25,99: 28,41; 28,93: 37,39: 38,07; 38,20) ($C\alpha$ à $C\eta$);
Partie sucre: 63,33 (C6); (62,65; 71,33; 72,69; 73,91) (C2,C3,C4,C5) (172,48; C1)
Fonction carboxylate: 177,37

**RMN $^1H$, DMSO $d_6$, $\delta$ppm:**

Chaine alkyle: 1,24 ($CH_2$: de $C_\gamma$ à $C_\varepsilon$); 1,42 ($CH_{2\beta}$); 1,9($CH_{2\tau}$); 3,5($CH_{2\alpha}$)
Partie sucre: 3-6(C$\underline{H}$OH, C$\underline{H}_2$OH); 7,66(N$\underline{H}$)

**Exemple 2:** Préparation du carboxylate de gluconamidodécane

On procède de la même manière que dans l'exemple 1 en utilisant 0,549 g d'acide aminoundécanoïque et 0,501 g de $\delta$-gluconolactone.

On récupère 1,02 g de composé de formule suivante:

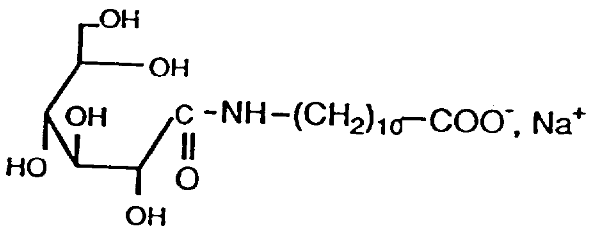

Ce bolaforme mixte est obtenu avec un rendement de 94,5 % et a été caractérisé par RMN, spectroscopie de masse et microanalyse.

**Exemple 3:** Mise en évidence des propriétés tensioactives du carboxylate de gluconamidodécane

Une étude par microscopie électronique de solutions de carboxylate de gluconamidodécane préparé selon l'exemple 2, a permis de mettre en évidence la présence de vésicules unilamellaires stables.

La technique de coloration négative a été utilisée selon le protocole suivant: quelques gouttes de la solution de bolaforme sont, après sonication, appliquées sur une grille de cuivre (150 mesh) recouverte d'un film de carbone. Le dépôt est desséché et une solution à 2% d'acétate d'uranyle dans l'eau est ensuite déposée de la même manière sur la grille.

Ces bolaformes mixtes chargés forment ainsi des vésicules unilamellaires stables et monodispersées (le diamètre des particules est compris entre 13 et 44 nm).

Les photographies des figures 1 et 2 ont été obtenues par microscopie électronique sur une solution de carboxylate de gluconamidodécane à une concentration de $5.10^{-2}$M.

**Exemple 4:** Préparation du carboxylate de gluconamidoundécane

Selon le même mode opératoire que dans l'exemple 1, avec 0,563 g d'acide amino-12-dodécanoïque et 0,490 g de $\delta$-gluconolactone, on obtient 95% de produit de formule suivante:

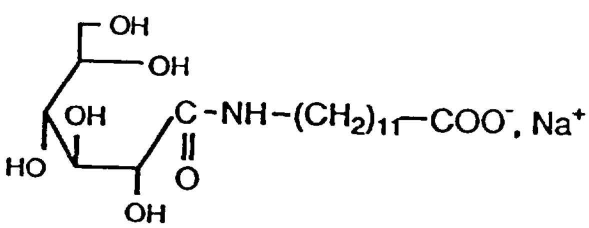

Ce composé a été obtenu avec un rendement de 94,5% et caractérisé par RMN, spectroscopie de masse et microanalyse.

**Exemple 5:** Préparation du carboxylate de lactobionamidopentane

A une solution de soude (2,7 mmoles: 0,110 g), sont ajoutés l'acide amino-5-pentanoïque (2,7 mmoles; 0,976 g) puis l'acide lactobionique. Le milieu réactionnel est agité pendant 24 heures dans le méthanol à 55°C. Le solvant est évaporé sous vide puis le produit obtenu est lyophilisé. On obtient 1,25 g de produit de formule suivante:

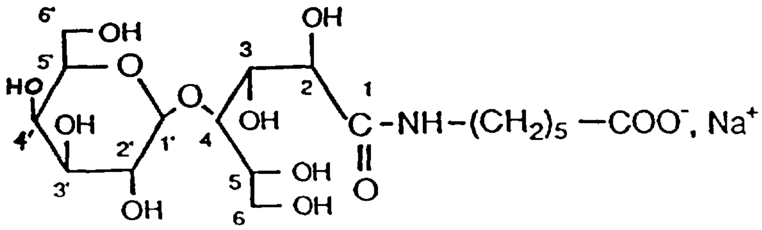

Ce nouveau bolaforme obtenu avec un rendement de 96% a été caractérisé par RMN $^1$H, RMN $^{13}$C, spectroscopie de masse et microanalyse.

**RMN $^{13}$C, DMSO $d_6$, $\delta$ ppm:**

Chaîne alkyle: $-NH-CH_2-CH_2-CH_2-CH_2-CH_2-COO^-, Na^+$

$\alpha$ $\beta$ $\gamma$ $\delta$ $\varepsilon$

(25,51; 26,29; 28,92; 37,15; 38,15) (C$\alpha$ à C$\varepsilon$) Partie sucre:
(C6:60,61; C6':62,10)
(66,16-82,61) (C2, C2', C3, C3', C4, C4', C5, C5') (C1:172,16; C1':104,56)
Fonction carboxylate: 177,38

**RMN $^1$H, DMSO $d_6$, $\delta$ ppm:**

| | |
|---|---|
| Chaîne alkyle: | ($CH_2\gamma$ :1,26); ($CH_2\beta$ :1,43); ($CH_2\delta$ :1,96) ($CH_2\alpha$, $CH_2\varepsilon$) (3-6) |
| Partie sucre: | (CHOH, $CH_2OH$) (3-6) |

**Exemple 6:** Préparation du carboxylate de lactobionamidoheptane

On procède de la même façon que dans l'exemple 5, avec 0,438 g d'acide amino-8-caprylique et 0,985 g d'acide lactobionique.

On récupère 1,37 g de produit de formule suivante:

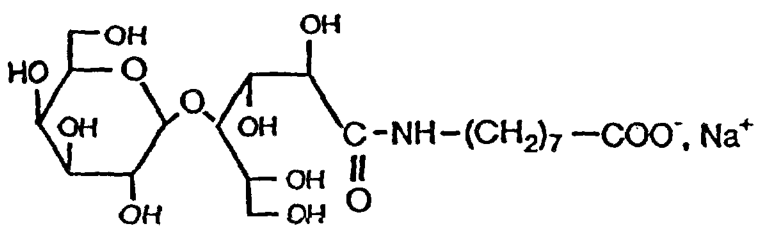

Ce produit obtenu avec un rendement de 98% a été caractérisé par RMN, spectroscopie de masse et microanalyse.

**Exemple 7:** Préparation du carboxylate de lactobionamidodécane

Selon le même protocole que dans l'exemple 5, avec 0,54 g d'acide aminoundécanoïque et 0,976 g d'acide lactobionique, on obtient 1,02 g de produit de formule suivante:

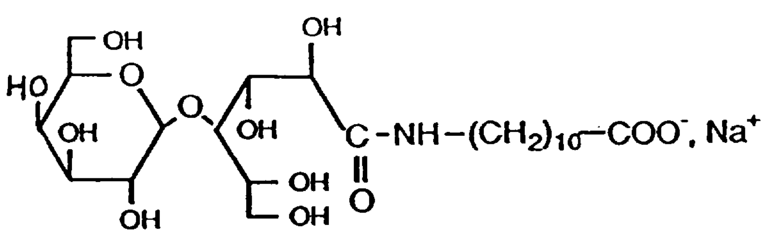

Le produit a été obtenu avec un rendement de 94,5% et caractérisé par RMN, spectroscopie de masse, microanalyse.

**Exemple 8:** Mise en évidence des propriétés tensioactives du carboxylate de lactobionamidodécane

Les propriétés tensioactives en solution aqueuse du carboxylate de lactobionamidodécane préparé suivant l'exemple 7 peuvent être mesurées par une technique usuelle de tensiométrie en utilisant un tensiomètre Tensimat N3 (Prolabo, FRANCE).

Le mobile de la mesure est un étrier fourni avec l'appareil formé d'un fil de platine de 0,1 mm de diamètre et long de 2 cm (écart entre les deux branches de l'étrier). Les résultats sont illustrés dans le tableau 1 indiquant les tensions superficielles (TS) à concentration variable en carboxylate de lactobionamidodécane.

Tableau 1:

| Pouvoir tensioactif du carboxylate lactobionamidododécane | |
|---|---|
| concentration | TS(mN/m) |
| $5.10^{-2}$ M | 40,65 |
| $10^{-2}$ M | 46,5 |
| $5.10^{-3}$ M | 54,65 |
| $10^{-3}$M | 62,85 |

**Exemple 9:** Préparation du carboxylate de lactobionamidoundécane

On procède de la même manière que dans l'exemple 5, avec 0,563 g d'acide amino-12-dodécanoïque et 0,985 g d'acide lactobionique et on récupère 1,59 g de produit de formule suivante:

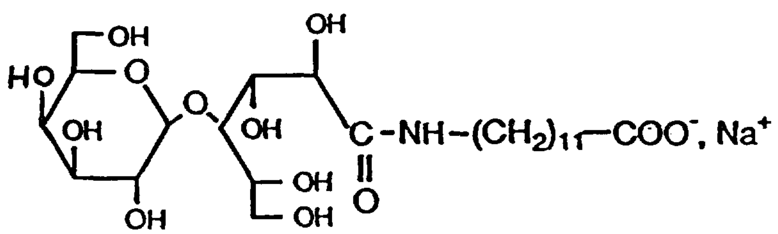

Ce bolaforme mixte est obtenu avec un rendement de 97% et a été caractérisé par RMN, spectroscopie de masse et microanalyse.

**Exemple 10:** Préparation du carboxylate dérivé de l'acide lactobionique

Cet exemple illustre la préparation de composés de formule I dans laquelle la chaîne carbonée E est interrompue par un groupe NHCO, à partir d'une anhydride, d'un acide diaminé de formule $NH_2$-E-$NH_2$ et d'une forme oxydée d'un glucide réducteur.

Les composés bolaformes précités répondent à la formule suivante:

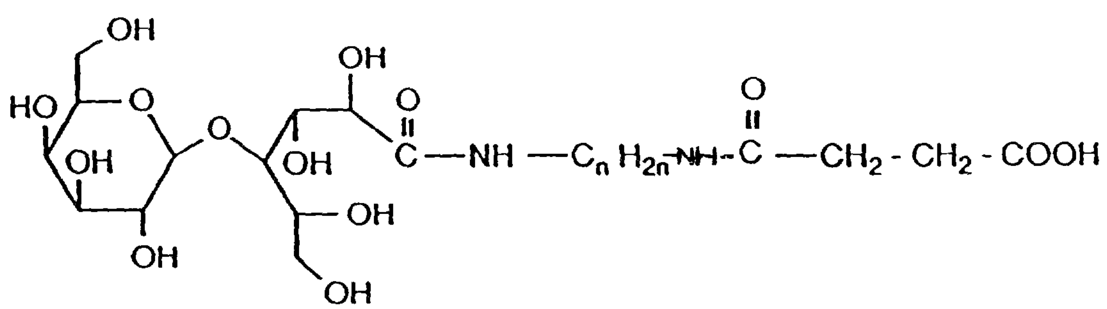

La chaîne carbonée de ces composés est interrompue par un groupe fonctionnel amide.

Ils sont synthétisés en deux étapes, la première étape consistant à préparer l'acide aminé de départ suivant:

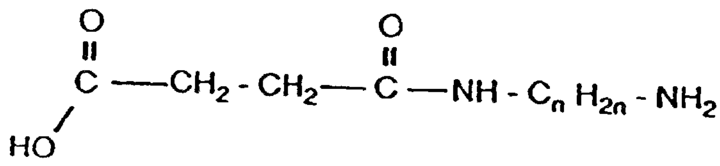

à partir de l'anhydride et de la diamine, selon la réaction suivante :

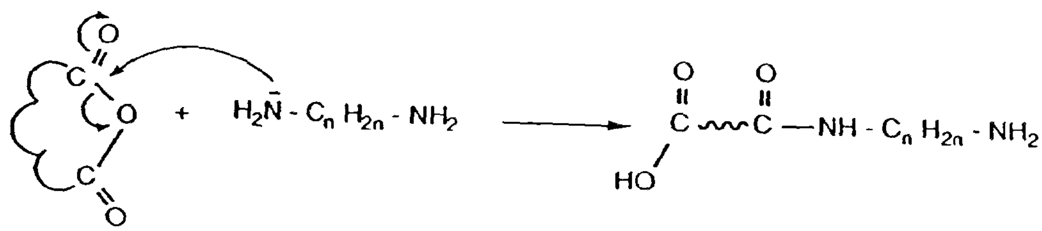

La réaction de la première étape n'est pas totale, et l'acide aminé obtenu est purifié par chromatographie sur colonne. L'anhydride, la diamine et l'acide aminé ayant des temps de rétention très distincts, la purification est facile.

Les acides aminés avec **n = 6** et **n = 12** sont obtenus, après purification avec respectivement des rendements de 31% et 38%.

Ils ont été caractérisés par RMN du $^1H$ et du $^{13}C$, microanalyse et spectrométrie de masse.

Lors de la deuxième étape, l'acide aminé est mis en solution dans du méthanol avec l'acide lactobionique.

A une solution contenant l'acide aminé (2,72 mmoles) dans du méthanol (100 ml), est ajouté l'acide lactobionique (2,72 mmoles) et l'hydroxyde de sodium (2,72 mmoles). Le mélange est mis sous agitation au reflux du méthanol pendant 6h puis 18h à température ambiante. Après évaporation du solvant, le bolaforme est purifié par chromatographie sur colonne de silice (MeOH: $HCCl_3$: $NH4^+OH^-$= 6: 3: 1 puis 4,5: 4,5: 1).

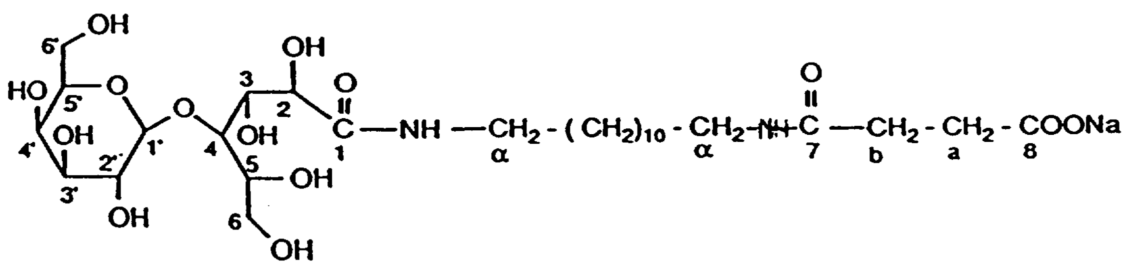

Le composé bolaforme avec n = 12 est obtenu, après purification avec un rendement de 45%. Il se présente sous forme de poudre blanche hygroscopique.

Ce dérivé a été caractérisé par RMN du $^1H$ et du $^{13}C$, microanalyse et spectrométrie de masse.

**RMN $^{13}C$, $CD_3OD$, δppm:**

- chaîne alkyle: 26-40,9 (10 $CH_2$, α + a + b $CH_2$); 172,1; 172,3 (C1, C7); 176(C8)
- partie sucre: 60,5; 62,3 (C6, C6'); 66-83 (C2, C2', C3, C3', C4, C4', C5, C5'); 104,5 (C1')

**RMN $^1H$, $CD_3OD$, δ ppm:**

1,36 (20H, m, $10CH_2$), 3 (4H, m, $\alpha CH_2$), 3,3-4,3 (25H, m, $aCH_2$ et $bCH_2$, C<u>H</u>O<u>H</u>, C<u>H</u>-O-C, $CH_2O$<u>H</u>)

**Masse:** FAB(-): 639 (M)

**Microanalyse:** $C_{28}H_{51}N_2O_{14}$ Na, $4H_2O$

| | | | | |
|---|---|---|---|---|
| % calculé: | C: 45,7, | H: 8,0, | N: 3,8, | O+Na: 42,3 |
| % trouvé: | 45,8 | 7,4 | 3,7 | 42,4 |

## Revendications

1. Composé chimique bolaamphiphile ou bolaforme, comportant une chaîne carbonée hydrophobe, linéaire ou ramifiée, aux extrémités de laquelle sont disposées deux parties ou têtes hydrophiles, ledit composé répondant à la formule générale suivante:

R A E Y

dans laquelle :

- E représente une chaîne carbonée, interrompue éventuellement par au moins un groupement aromatique, hétérocyclique, ou fonctionnel
- A est un groupe fonctionnel
- R est un résidu d'un glucide réducteur à chaîne linéaire ou cyclisée, ou un dérivé du glucide réducteur, lié directement ou indirectement par une de ses fonctions aldéhyde ou cétone, ou dérivée, au groupe fonctionnel A,
- Y représente une tête hydrophile,

**caractérisée en ce que** Y comprend au moins un groupe anionique choisi parmi les ions carboxylate, sulfate, phosphate, sulfonate, phosphonate, Y étant lié directement ou indirectement à la chaîne carbonée E.

**2.** Composé selon la revendication 1, caractérisé en ce que le glucide réducteur est un monosaccharide.

**3.** Composé selon la revendication 2, caractérisé en ce que le monosaccharide est choisi dans le groupe comprenant le glucose et le galactose.

**4.** Composé selon la revendication 1, caractérisé en ce que le glucide réducteur est un polysaccharide.

**5.** Composé selon la revendication 4, caractérisé en ce que le polysaccharide est un disaccharide, notamment choisi dans le groupe comprenant le lactose, le cellobiose et le maltose.

**6.** Composé selon la revendication 1, caractérisé en ce que la chaîne carbonée hydrophobe est une chaîne carbonée saturée.

**7.** Composé selon la revendication 6, caractérisé en ce que la chaîne carbonée comprend jusqu'ã 12 atomes de carbone.

**8.** Composé selon la revendication 1, caractérisé en ce que le groupe fonctionnel A est choisi dans le groupe comprenant les fonctions :

O, OCO, S, NH, NHCO

**9.** Composé selon la revendication 8, caractérisé en ce que le groupe fonctionnel A est NH.

**10.** Composé selon la revendication 3 ou 5, caractérisé en ce que Y est un groupe anionique carboxylate.

**11.** Composé selon la revendication 1, caractérisé en ce que Y est indirectement lié à la chaîne carbonée E, par l'intermédiaire d'un groupe fonctionnel choisi parmi les fonctions O, OCO, S, NH, NHCO.

**12.** Composé selon l'une quelconque des revendications 1 à 11, caractérisé en ce qu'il est obtenu par mise en réaction d'une forme oxydée, ou réduite, ou aminée du glucide réducteur, avec un produit répondant à la formule B E Y', B correspondant au groupe fonctionnel A avant condensation avec la forme réactive du glucide, et Y' correspondant à la forme non chargée du groupe Y, en présence d'une base.

**13.** Composé selon la revendication 12, caractérisé en ce que la forme oxydée du glucide réducteur est la forme lactone ou acide.

**14.** Composé selon la revendication 3 et la revendication 13, caractérisé en ce que la forme lactone du glucide réducteur est la gluconolactone.

**15.** Composé selon la revendication 5 et la revendication 13, caractérisé en ce que la forme acide du glucide réducteur est l'acide lactobionique.

**16.** Composé selon la revendication 10 et selon la revendication 14 ou 15, caractérisé en ce qu'il est obtenu par mise en réaction directement de la gluconolactone ou l'acide lactobionique, respectivement, sur un acide aminé répondant à la formule BEY', Y' correspondant à YH et représentant la fonction acide dudit acide aminé, en présence de soude.

**17.** Composé selon la revendication 10 et la revendication 16, caractérisé en ce que ledit acide aminé est choisi parmi l'acide amino-6-hexanoïque, l'acide amino-8-caprylique, l'acide amino-11-undécanoïque, et l'acide amino-12-dodécanoïque.

**18.** Composé selon la revendication 16, caractérisé en ce que ledit acide aminé répond à la formule BEY', dans laquelle E est une chaîne carbonée interrompue par un groupe NHCO.

**19.** Composé selon la revendication 18, caractérisé en ce que ledit acide aminé est obtenu par réaction d'une anhydride avec un acide diaminé répondant à la formule :

$$NH_2\text{-}E\text{-}NH_2$$

**20.** Composition tensloactive caractérisée en ce qu'elle incorpore au moins un composé selon l'une quelconque des revendications 1 à 19.

**21.** Utilisation d'un composé répondant à la formule générale suivante :

R A E Y

dans laquelle :

- E représente la chaîne carbonée, interrompue éventuellement par au moins un groupement aromatique, hétérocyclique, ou fonctionnel
- A est un groupe fonctionnel
- R est un résidu d'un glucide réducteur à chaîne linéaire ou cyclisée, ou un dérivé du glucide réducteur, lié directement ou indirectement par une de ses fonctions aldéhyde ou cétone, ou dérivée, au groupe fonctionnel A,
- Y représente une tête hydrophile,

comportant au moins un groupe anionique choisi parmi les ions carboxylate, sulfate, phosphate et phosphonate, Y étant lié directement ou indirectement à la chaîne carbonée E,
pour l'obtention d'un agent tensioactif.

**22.** Agent vésiculaire, caractérisé en ce qu'il comprend au moins un composé selon l'une quelconque des revendications 1 à 19.

FIG 1

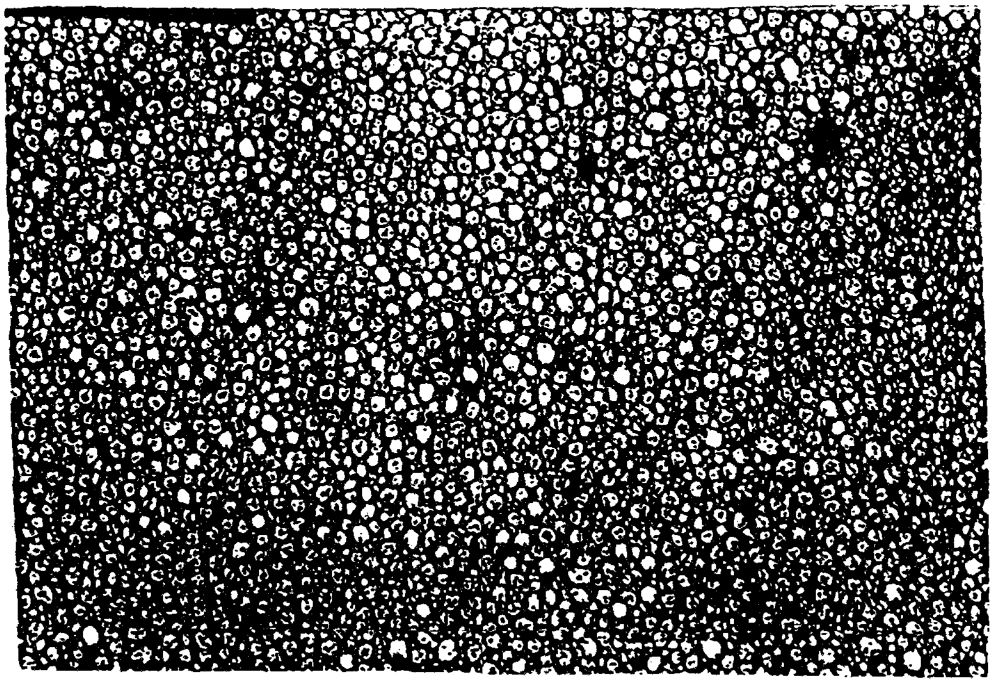

FIG 2

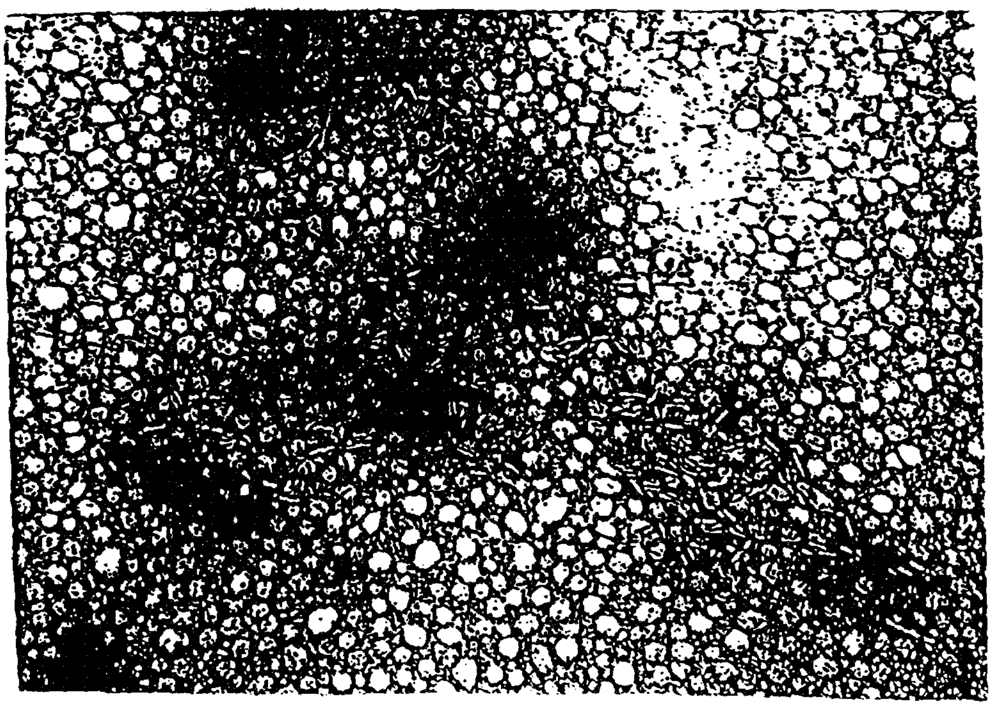

Office européen des brevets

# RAPPORT DE RECHERCHE EUROPEENNE

Numero de la demande
EP 95 42 0311

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | LANGMUIR, vol. 6, no. 2, Février 1990 pages 497-505, J. FUHRHOP ET AL 'Bolaform amphiphiles with a rigid hydrophobic bixin core in surface monolayers and lipid membranes.' * page 497 - page 498 * | 1-22 | C07H15/06 C07H15/04 C07H15/12 C07H13/04 C07C235/10 C11D1/00 A61K9/127 B01F17/56 |
| Y | CHEMISTRY AND PHYSICS OF LIPIDS, vol. 43, 1987 pages 193-213, J. FUHRHOP & H. TANK 'Bolaamphiphiles with mannose- and tetraalkylammonium head groups as coatings for nucleic acids and possible reagents for transfections.' * page 195 * | 1-22 | |
| Y | FR-A-2 523 962 (CENTRE NATIONAL DE LA RECHERCHE SCIENTIFIC, CNSC) 30 Septembre 1983 * page 1 * | 1-22 | |
| | | | DOMAINES TECHNIQUES RECHERCHES (Int.Cl.6) |
| D,A | EP-A-0 541 467 (STEPAN EUROPE) 12 Mai 1993 * le document en entier * | 1,20-22 | C07H C07C C11D A61K B01F |
| A | SYNTHETIC COMMUNICATIONS, vol. 23, no. 1, 1993 pages 35-44, R. GARELLI-CALVET ET AL 'Bis-glucoamides and bis-lactobioamides novel alpha,omega-type (bolaform) surfactants with two sugar head groups.' * le document en entier * | 1,20-22 | |

-/--

Le présent rapport a été établi pour toutes les revendications

| Lieu de la recherche | Date d'achèvement de la recherche | Examinateur |
|---|---|---|
| LA HAYE | 7 Février 1996 | Moreno, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 95 42 0311

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | ANGEWANDTE CHEMIE INTERNATIONAL EDITION., vol. 23, 1984 WEINHEIM DE, pages 100-113, J. FUHRHOP & J. MATHIEU 'Routes to functional vesicle membranes without proteins.' * le document en entier * ----- | 1,20-22 | |

DOMAINES TECHNIQUES RECHERCHES (Int.Cl.6)

Le présent rapport a été établi pour toutes les revendications

| Lieu de la recherche | Date d'achèvement de la recherche | Examinateur |
|---|---|---|
| LA HAYE | 7 Février 1996 | Moreno, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)